Europäisches Patentamt

European Patent Office

Office européen des brevets

(19)

(11) Veröffentlichungsnummer : **0 215 468**
**B1**

(12)　　　　　　**EUROPÄISCHE PATENTSCHRIFT**

(45) Veröffentlichungstag der Patentschrift :
**13.12.89**

(21) Anmeldenummer : **86112731.4**

(22) Anmeldetag : **15.09.86**

(51) Int. Cl.⁴ : **A 61 F 2/16**, A 61 F 2/14

(54) **Künstliche Linse zur Implantation im Auge.**

(30) Priorität : **14.09.85 DE 3532928**

(43) Veröffentlichungstag der Anmeldung :
**25.03.87 Patentblatt 87/13**

(45) Bekanntmachung des Hinweises auf die Patenterteilung : **13.12.89 Patentblatt 89/50**

(84) Benannte Vertragsstaaten :
**BE CH DE FR GB LI NL**

(56) Entgegenhaltungen :
**WO–A–84 /048 81**
**US–A– 4 426 741**
**US–A– 4 588 405**
**US–A– 4 610 689**

(73) Patentinhaber : **Fromberg, Gunther, Dr.**
**Brühlstrasse 2**
**D-6690 St. Wendel (DE)**

**Walter, John**
**Brühlstrasse 2**
**D-6690 St. Wendel (DE)**

(72) Erfinder : **Fromberg, Gunter, Dr.**
**Brühlstrasse 2**
**D-6690 St. Wendel (DE)**

(74) Vertreter : **Bernhardt, Winfrid, Dr.-Ing.**
**Kobenhüttenweg 43**
**D-6600 Saarbrücken (DE)**

EP 0 215 468 B1

## Beschreibung

Die Erfindung betrifft eine einteilige künstliche Linse zur Implantation im Auge mit Haltebügeln zur Abstützung der Linse im Vorder- oder Hinterkammerwinkel oder im Kapselsack, die sich im wesentlichen parallel zur Linsenebene vom Umfang der Linse aus nach außen erstrecken und bis zu ihrem freien Ende nach der Umfangsrichtung und vorzugsweise über diese hinaus gebogen sind.

Solche künstlichen Linsen aus Kunststoff werden bei der Operation des Grauen Stars zum Ersatz für die entfernte natürliche Augenlinse implantiert.

Der Erfindung liegt die Aufgabe zugrunde, eine künstliche Linse von größerer Verträglichkeit zu schaffen als sie bisher zur Verfügung steht.

Gemäß der Erfindung erfüllt diesen Zweck eine Linse der eingangs bezeichneten Art, die aus Silikonkautschuk besteht und deren Bügel auf einem äußeren Abschnitt ihrer Länge einen Querschnitt mit einer zu der Linsenebene im wesentlichen parallelen Begrenzung und einer über dieser gekrümmten oder gewinkelten Begrenzung und eine Breite von 0,6 bis 1,5 mm aufweisen und sich auf einem inneren Abschnitt ihrer Länge mindestens auf das 1,5-fache verbreitern.

Während bei den bisherigen künstlichen Linsen an den Bügeln Druckkräfte von 0,5 N bis 10 N und mehr für 0,5 mm Auslenkung auftreten, sind es bei der neuen Linse nur noch 20 bis 30 mN. Trotzdem ist die neue Linse bei der vorgesehenen Ausbildung und Bemessung der Bügel, insbesondere ihrer Verbreiterung auf dem inneren Abschnitt, sicher gehalten, zumal sie bei ihrem geringen spezifischen Gewicht in dem Kammerwasser fast schwebt. Sie hat sogar, anders als die meisten bekannten künstlichen Linsen, keine Neigung, aus ihrer vorgesehenen Ebene herauszuspringen. Infolge der Verbreiterung der Bügel auf dem inneren Abschnitt ist hier eine Biegesteifigkeit vorhanden, die eine Verwinkelung der Bügel an ihrem inneren Ende gegenüber dem Linsenkörper, wie sie mit dem Herausspringen der Linse aus der vorgesehenen Ebene zwangsläufig verbunden ist, verhindert; die Bügel gehen in den Linsenkörper über. Auch die vorgesehene Verbiegung der Bügel innerhalb ihrer Ebene beschränkt sich auf ihren äußeren Abschnitt.

Die Materialauswahl des Silikonkautschuks auch für die Bügel erlaubt aufgrund der Nachgiebigkeit dieses Materials die für die Bügel vorgesehene Querschnittsform, deren genannte, zu der Linsenebene im wesentlichen parallele Begrenzung eine flächige Anlage der Bügel an der Regenbogenhaut bildet und deren darüber gekrümmte und/oder gewinkelte Begrenzung gleichfalls eine verhältnismäßig breitflächige Anlage im Kammerwinkel des Auges bildet im Vergleich zu den bekannten Bügeln, die demgegenüber als dünne Drähte von rundem Querschnitt auf geringeren, mehr oder weniger linienförmigen Anlageflächen und überdies mit weitaus größerer Kraft in den Kammerwinkel drücken. Die breitflächige Anlage im Kammerwinkel ergibt sich bei der gekrümmten Begrenzung aus der vorgesehenen Breite der Bügel. Bei der gewinkelten Begrenzung, die mit der zur Linsenebene parallelen Begrenzung z. B. ein flaches Dreieck o. ä. bildet, kann sie noch flacher und breiter sein.

So ist die neue Linse unvergleichlich schonender und verträglicher, sie ruft keine Druckatrophien hervor. Auch in den Kapselsack läßt sie sich gut einsetzen.

Darüberhinaus verlangt die neue Linse bei der Operation nur einen kleineren Schnitt, da sie gefaltet in das Auge eingeführt werden kann. Auch kann sie mit einfacheren Instrumenten implantiert werden. Schließlich ist von Vorteil, daß sowohl das Material als auch die einteilige Herstellung im Spritzgußverfahren kostengünstig ist.

Die Gegenstände der Unteransprüche stellen Optimierungen unter den vorstehenden Gesichtspunkten dar.

Die Zeichnungen geben Ausführungsbeispiele der Erfindung wieder.

Fig. 1 zeigt eine erfindungsgemäße Linse in Draufsicht,

Fig. 2 zeigt die Linse nach Fig. 1 links im Querschnitt und rechts in Seitenansicht,

Fig. 3 zeigt eine weitere erfindungsgemäße Linse in Draufsicht,

Fig. 4 zeigt die Linse nach Fig. 3 im Querschnitt.

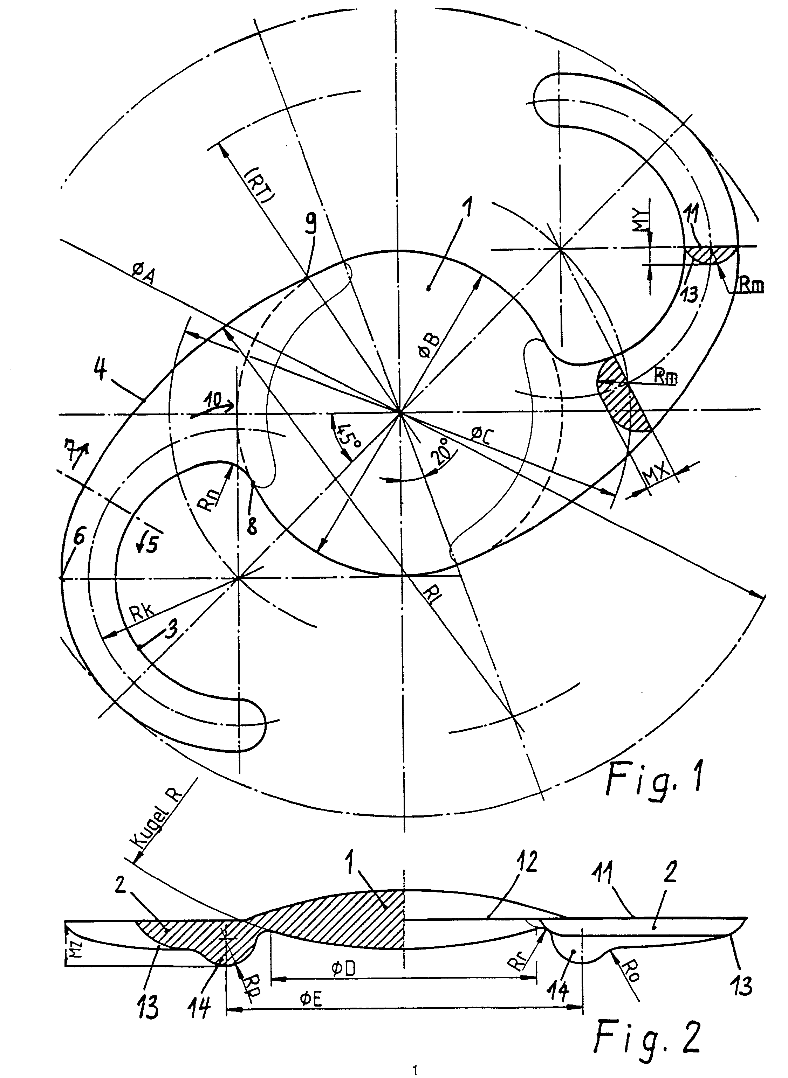

Die Linse nach Fig. 1 weist an einem biconvexen Linsenkörper 1 in symmetrischer Anordnung zwei Bügel 2 auf.

Die Bügel 2 sind an ihrem inneren Rand 3 im wesentlichen halbkreisbogenförmig. Ihr äußerer Rand 4 verläuft auf einem äußeren Abschnitt der Bügel von etwa 130° Bogenlänge parallel zum inneren Rand 3 und legt sich dann mit einer leichten Krümmung etwa tangential an den Linsenumfang an.

Damit gliedern sich die Bügel 2 in einen gleichbleibenden, kreisbogenförmigen äußeren Abschnitt 5 von etwa 130° Bogenlänge, der um etwa 45° Bogenlänge über den äußersten Punkt 6 hinausgeht, und einen sich verbreiternden inneren Abschnitt 7 von etwa 50° Bogenlänge (gemessen in der bogenförmigen Verlängerung des äußeren Abschnitts 5).

Diese Bemessungen könnten auch etwas abgewandelt werden. Allerdings sollte der äußere Abschnitt 5 mindestens einen Viertelkreisbogen betragen und der innere Abschnitt 7 sich mindestens an 1/5 und höchstens etwa an 1/3 der Länge des inneren Randes 3 des Bügels erstrecken.

An ihrem inneren Ende verbreitern sich die Bügel 2 verstärkt. Einerseits ergibt sich dies aus dem etwa tangentialen Anschluß des äußeren Randes 4 an den Linsenumfang. Auf der anderen Seite ist das innere Ende des sonst kreisbogenförmigen inneren Randes 3 ausgewölbt (Radius Rn). Der Anschluß der Bügel 2 an dem Linsenkörper 1 erstreckt sich jeweils über fast 1/4 der Umfangslänge des Linsenkörpers. Hier dürften Varianten von 1/5 bis 1/3 der Umfangslänge in Betracht kommen.

Betrachtet man diesen Anschluß der Bügel an dem Linsenkörper bezogen auf die Verbindungslinie des Linsenmittelpunktes mit dem äußersten Punkt 6, an dem jeweils im Mittel die Haltekraft an dan Bügeln angreift, so reicht er von einem etwa 20° von dieser Linie entfernten Punkt 8 bis zu einem etwas hinter dem seitlichen Extrempunkt liegenden Punkt 9 des Linsenumfanges, und so dürfte der Schwerpunkt des Kräfteflusses etwa an dem Pfeil 10 in etwa 45° Winkelabstand von dieser Linie liegen.

Die Breite der Bügel 2 beträgt auf dem äußeren Abschnitt 5 0,9 mm. Der Querschnitt hat die Form eines Kreisabschnitts, wie in Fig. 1 oben rechts eingezeichnet. Als Variationsbreite kommt hier 0,8 bis 1,1 mm, vorzugsweise 0,85 bis 0,95 mm, in engerer Wahl in Betracht. Die Querschnittsform könnte auch bis zum Halbkreis oder etwas darüber reichen.

Die Bügel verbreitern sich dann auf dem inneren Abschnitt 7 bis zu der genannten verstärkten Verbreiterung etwa auf das 2-fache. Dazu sei eine Variationsbreite vom 1,8- bis 2,5-fachen angegeben. Wie der in Fig. 1 rechts eingezeichnete Querschnitt zeigt, verdicken sich die Bügel dabei auch.

Die eine, ebene, Oberfläche der Bügel 2, die mit 11 bezeichnet ist, liegt in der Hauptschnittebene 12 der Biconvexlinse, siehe Fig. 2.

Die andere, gewölbte, Oberfläche der Bügel 2 ist mit 13 bezeichnet. Auf dieser erhebt sich am inneren Ende der Bügel jeweils eine Aufwölbung 14 als « laser spacer », wie an sich bekannt.

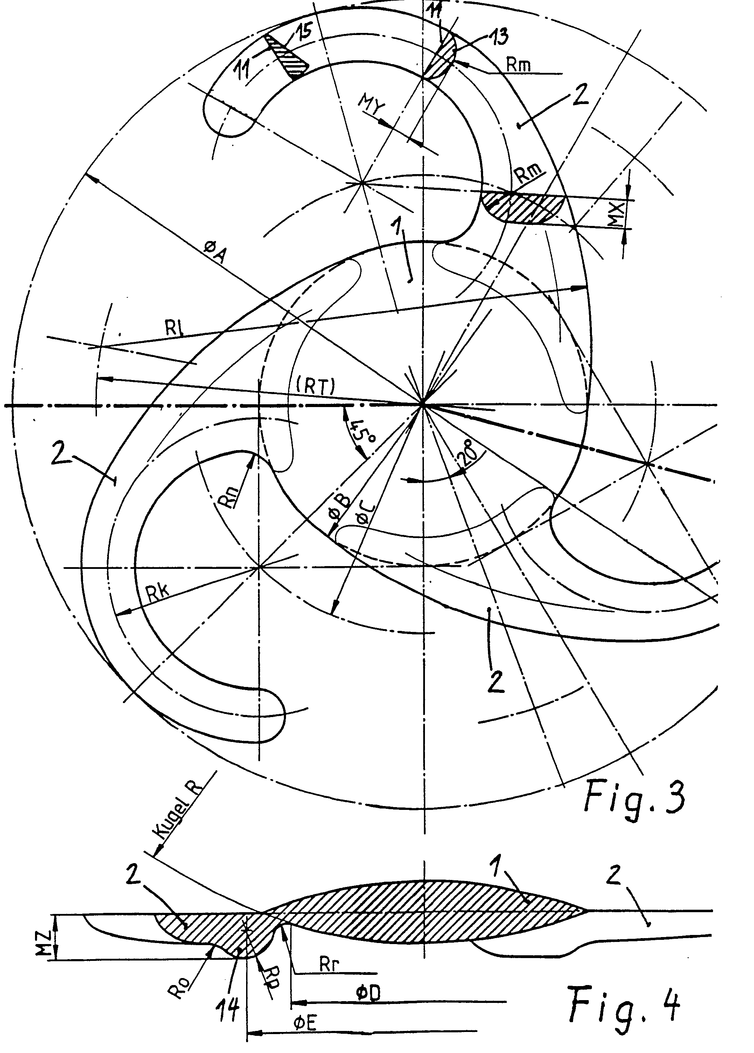

Die vorstehenden Beschreibungen gelten für die Linse nach Fig. 3 und 4 in gleicher Weise mit der Ausnahme, daß diese Linse statt zwei Bügeln 2 drei Bügel 2 in drehsymmetrischer Anordnung aufweist und der Ansatz der Bügel am Linsenumfang geringfügig kürzer ist.

Als andere Variante des Bügelquerschnitts ist in Fig. 3 oben ein Querschnitt eingezeichnet, der über der ebenen Oberfläche 11 statt der gekrümmten Oberfläche 13 eine gewinkelte Oberfläche mit einer ebenen Anlagefläche 15 zur Abstützung im Vorder- oder Hinterkammerwinkel des Auges aufweist. Die Winkel, besonders derjenige zwischen der ebenen Oberfläche 11 und der Anlagefläche 15, sind gerundet.

Die in den Zeichnungen angegebenen Größen betragen in mm : ØA : 13,75 ; ØB : 5,5 ; ØC : 7,77 ; ØD : 4,5 ; ØE : 6 ; Rk : 2,52 ; Rl : 8,25 ; Rm : 0,5 ; Rn : 0,6 ; Ro : 0,5 ; Rp : 0,5 ; Rr : 0,25 ; RKugel : 7,643 ; (RT) : 11 ; MX : 0,5 ; MY : 0,3 ; MZ : 0,76.

Die beiden Linsen sind sowohl als Vorderkammerlinse als auch als Hinterkammerlinse einsetzbar.

Der Silikonkautschuk ist ein von Säuren, Katalysatoren und sonstigen nicht in das Polymerisat chemisch eingebundenen niedermolekularen Stoffen gereinigter Silikonkautschuk mit einer Härte Shore A von etwa 46 und einer Dichte von etwa 1,04 g/cm$^3$.

## Patentansprüche

1. Einteilige Linse (1) zur Implantation im Auge mit Haltebügeln (2) zur Abstützung der Linse im Vorder- oder Hinterkammerwinkel oder im Kapselsack, die sich im wesentlichen parallel zur Linsenebene (12) vom Umfang der Linse aus nach außen erstrecken und bis zu ihrem freien Ende nach der Umfangsrichtung und vorzugsweise über diese hinaus gebogen sind, dadurch gekennzeichnet, daß die Linse (1) aus Silikonkautschuk besteht und die Bügel (2) auf einem äußeren Abschnitt (5) ihrer Länge einen Querschnitt mit einer zu der Linsenebene (12) im wesentlichen parallelen Begrenzung (11) und einer über dieser gekrümmten und/oder gewinkelten Begrenzung (13, 15) und eine Breite von 0,6 bis 1,5 mm aufweisen und sich auf einem inneren Abschnitt (7) ihrer Länge mindestens auf das 1,5-fache verbreitern.

2. Linse nach Anspruch 1, dadurch gekennzeichnet, daß der genannte äußere Abschnitt (5) im wesentlichen kreisbogenförmig ist und die Länge mindestens eines Viertelkreisbogens hat und die Biegung etwa um einen Achtelkreisbogen über den äußersten Punkt (6) hinausgeht.

3. Linse nach Anspruch 1 oder 2, dadurch gekennzeichnet, daß die Bügel (2) im ganzen an ihrem inneren Rand (3) im wesentlichen halbkreisbogenförmig sind und der genannte innere Abschnitt (7) sich an 1/5 bis 1/3, vorzugsweise etwa 1/4, der Länge dieses Halbkreisbogens erstreckt.

4. Linse nach einem der Ansprüche 1 bis 3, dadurch gekennzeichnet, daß die Bügel (2) sich an ihrem inneren Ende verstärkt verbreitern und am Linsenumfang 1/5 bis 1/3, vorzugsweise etwa 1/4, von dessen Länge einnehmen, vorzugsweise mit Schwerpunkt (10) in einem auf den Linsenmittelpunkt bezogenen Winkelabstand von etwa 45° von dem äußersten Punkt (6) des Bügels.

5. Linse nach den Ansprüchen 3 und 4, dadurch gekennzeichnet, daß der äußere Rand (4) der Bügel (2), vorzugsweise mit einer leichten Krümmung, etwa tangential an den Linsenumfang anschließt (Punkt 9).

6. Linse nach Anspruch 4 oder 5, dadurch gekennzeichnet, daß die Bügel (2) sich bis zu der genannten verstärkten Verbreiterung auf das 1,8- bis 2,5-fache, vorzugsweise etwa das 2-fache, verbreitern.

7. Linse nach einem der Ansprüche 1 bis 6, dadurch gekennzeichnet, daß die Breite der Bügel (2) auf dem genannten äußeren Abschnitt (5) 0,8 bis 1,1 mm, vorzugsweise 0,85 bis 0,95 mm, beträgt.

8. Linse nach einem der Ansprüche 1 bis 7, dadurch gekennzeichnet, daß der Querschnitt der Bügel (2) auf dem genannten äußeren Abschnitt (5) halbkreisförmig oder knapp halbkreisförmig ist.

9. Linse nach einem der Ansprüche 1 bis 8, dadurch gekennzeichnet, daß sie eine Biconvexlinse ist und die genannte zur Linsenebene parallele Begrenzung (11) des Bügelquerschnitts in der Hauptschnittebene (12) der Biconvexlinse liegt.

10. Linse nach einem der Ansprüche 1 bis 9, dadurch gekennzeichnet, daß sie zwei oder drei Bügel (2) in drehsymmetrischer Verteilung aufweist.

11. Linse nach einem der Ansprüche 1 bis 10, dadurch gekennzeichnet, daß der Silikonkautschuk ein von Säuren, Katalysatoren und sonstigen nicht in das Polymerisat chemisch eingebundenen niedermolekularen Stoffen gereinigter Silikonkautschuk mit einer Härte Shore A von 30 bis 60, vorzugsweise 40 bis 50, und einer Dichte von 1,02 bis 1,07, vorzugsweise 1,03 bis 1,05, g/cm³ ist.

## Claims

1. A single-part lens (1) for implantation in the eye having supports (2) for supporting the lens in the anterior or posterior chamber angle or in the capsular bag, which extend substantially parallel to the plane of the lens (12) from the circumference of the lens towards the outside and are bent right up to their free ends in the circumferential direction and preferably past them, characterised in that the lens (1) is made from silicon rubber and on an outer section (5) of their length the supports (2) have a cross section with a boundary (11) which is substantially parallel to the plane of the lens (12) and with a boundary (13, 15) curved and/or angled over this and have a width of 0.6 to 1.5 mm and on an inner section (7) of their length widen at least by 1.5 times.

2. A lens according to Claim 1, characterised in that said outer section (5) is substantially circular and its length is at least one fourth part of the circumference and the curvature extends past the outermost point (6) by approximately one eighth part of the circumference.

3. A lens according to Claim 1 or 2, characterised in that overall the supports (2) are substantially semi-circular on their inner edge (3) and said inner section (7) extends to 1/5 to 1/3, preferably approximately 1/4, the length of this semi-circle.

4. A lens according to Claims 1 to 3, characterised in that the supports (2) become much wider at their inner end and at the lens circumference occupy 1/5 to 1/3, preferably approximately 1/4, of its length, preferably with the centre of mass (10) at an angular distance of approximately 45° from the outermost point (6) of the support with respect to the centre point of the lens.

5. A lens according to Claims 3 and 4, characterised in that the outer edge (4) of the supports (2) is connected approximately tangentially to the circumference of the lens (point 9), preferably with a slight curvature.

6. A lens according to Claim 4 or 5, characterised in that the supports (2) become wider right up to said much wider section by 1.8 to 2.5 times, preferably approximately 2 times.

7. A lens according to one of Claims 1 to 6, characterised in that the width of the supports (2) on said outer section (5) is 0.8 to 1.1 mm, preferably 0.85 to 0.95 mm.

8. A lens according to one of Claims 1 to 7, characterised in that the cross section of the supports (2) on said outer section (5) is semi-circular or almost semi-circular.

9. A lens according to one of Claims 1 to 8, characterised in that it is a biconvex lens and said boundary (11) of the support cross section, which is parallel to the plane of the lens, lies in the plane of the principal section (12) of the biconvex lens.

10. A lens according to one of Claims 1 to 9, characterised in that it has two or three supports (2) which are distributed with rotational symmetry.

11. A lens according to one of Claims 1 to 10, characterised in that the silicon rubber is a silicon rubber which is cleaned by acids, catalysts and other low-molecular substances not chemically bound into the polymeride and which has a Shore hardness A of 30 to 60, preferably 40 to 50, and a density of 1.02 to 1.07, preferably 1.03 to 1.05 g/cm³.

## Revendications

1. Lentille d'une seule pièce (1) pour implantation dans l'œil, comportant, pour l'appui de la lentille dans l'angle avant ou arrière du logement ou dans la capsule, des étriers de maintien (2) qui s'étendent sensiblement parallèlement au plan (12) de la lentille depuis la périphérie de la lentille vers l'extérieur et, jusqu'à leur extrémité libre, sont incurvés dans la direction périphérique et de préférence au-delà, caractérisée en ce que la lentille (1) est en caoutchouc de silicone et que les étriers présentent, sur une partie extérieure (5) de leur longueur, une section transversale comportant une surface de délimitation (11) sensiblement parallèle au plan (12) de la lentille et une surface de délimitation (13, 15) incurvée et/ou pliée située en face, et ont une largeur de 0,6 à 1,5 mm et, sur une partie intérieure (7) de leur longueur, s'élargissent de 1,5 fois au moins.

2. Lentille selon la revendication 1, caractérisée en ce que la partie extérieure (5) a sensiblement la forme d'un arc de cercle dont la longueur est d'au moins un quart de circonférence et que l'incurvation se prolonge au moins d'un arc de huitième de circonférence au-delà du point le plus extérieur (6).

3. Lentille selon l'une des revendications 1 ou 2, caractérisée en ce que les étriers (2) présentent dans leur ensemble au niveau de leur bord intérieur (3) une forme semi-circulaire et que la partie intérieure (7) s'étend sur 1/5 à 1/3, de préférence sur le 1/4 environ de la longueur de cette demi-circonférence.

4. Lentille selon une des revendications 1 à 3, caractérisée en ce que les étriers (2) s'élargissent beaucoup à leur extrémité intérieure et occupent de 1/5 à 1/3, de préférence le 1/4 de la longueur du périmètre de la lentille, de préférence de telle manière que le centre de gravité (10) se trouve à une distance angulaire, rapportée au centre de la lentille, de 45° du point le plus extérieur (6) de l'étrier.

5. Lentille selon les revendications 3 et 4, caractérisée en ce que le bord extérieur (4) des étriers (2) se raccorde à peu près tangentiellement, de préférence avec une légère incurvation, à la périphérie de la lentille (point 9).

6. Lentille selon une des revendications 4 ou 5, caractérisée en ce que les étriers (2) s'élargissent, jusqu'à la partie élargie renforcée, de 1,8 à 2,5 fois, de préférence de 2 fois.

7. Lentille selon une des revendications 1 à 6, caractérisée en ce que la largeur des étriers (2) dans la partie extérieure (5) est de 0,8 à 1,1 mm, de préférence de 0,85 à 0,95 mm.

8. Lentille selon une des revendications 1 à 7, caractérisée en ce que la section transversale des étriers (2) dans la partie extérieure (5) est semi-circulaire ou presque semi-circulaire.

9. Lentille selon une des revendications 1 à 8, caractérisée en ce qu'elle est une lentille biconvexe et que la surface de délimitation (11) de la section transversale de l'étrier parallèle au plan de la lentille se trouve dans le plan (12) de la section principale de la lentille biconvexe.

10. Lentille selon une des revendications 1 à 9, caractérisée en ce qu'elle comporte deux ou trois étriers (2) disposés symétriquement par rapport à un centre de rotation.

11. Lentille selon une des revendications 1 à 10, caractérisée en ce que le caoutchouc de silicone est un caoutchouc de silicone débarrassé par purification d'acides, de catalyseurs et d'autres substances de faible poids moléculaire non liées chimiquement dans le produit de polymérisation et ayant une dureté Shore A de 30 à 60, de préférence de 40 à 50, et une densité de 1,02 à 1,07, de préférence de 1,03 à 1,05 g/cm$^3$.

EP 0 215 468 B1

Fig. 1

Fig. 2

Fig. 3

Fig. 4